# EUROPEAN PATENT SPECIFICATION

(11) **EP 1 433 443 B1**
(45) Date of publication and mention of the grant of the patent: **12.07.2006**
(21) Application number: 03257871.8
(22) Date of filing: 15.12.2003
(51) Int. Cl.: A61F 2/32, A61F 2/30

(54) **Hip prosthesis with ceramic bearing**
Hüftprothese mit keramischem Lager
Prothèse de hanche avec palier en céramique

(30) Priority: 27.12.2002 GB 0230214
(43) Date of publication of application: 30.06.2004
(73) Proprietor: Biomet Merck Limited, Bridgend, South Wales CF31 3XA (GB)
(72) Inventor: Bigsby, Robert John Andrew, Penarth Glamorgan CF64 1EJ (GB); Scott, Robert Andrew, Chippenham Wiltshire SN15 4BX (GB)
(74) Representative: Giles, Ashley Simon

(56) References cited:
- EP-A- 0 202 141
- EP-A- 0 278 205
- EP-A- 1 290 992
- WO-A-98/42390
- FR-A- 2 310 120
- US-A- 5 716 414
- US-B1- 6 319 285

## Description

This invention relates to a hip prosthesis comprising a metal support permanently bonded to a ceramic bearing.

### BACKGROUND TO THE INVENTION

Ceramic acetabular cups were first used in 1970 by the French surgeon, Pierre Boutin. He used 32mm diameter alumina heads combined with cemented alumina cups. One of the main problems with this design was obtaining satisfactory fixation in the acetabulum with these non-modular ceramic shells. A cementless acetabular component was introduced in 1972 by Mittelmeier in Germany. His design consisted of a monolithic ceramic, externally threaded truncated cone socket which was screwed into an under-reamed acetabulum to articulate with a 32 or 38 mm spherical head mounted on a cementless stem. The ceramic screw-in cup design was non-modular, the results were generally not successful.

Subsequent designs of ceramic cups have almost universally been of a modular construction. This generally takes one of two forms. Firstly, the cup can be in the form of a ceramic insert fitting in a tapered recess in a metal shell. Alternatively, it can be comprised of a ceramic insert moulded or press fitted into a polyethylene liner, which is then pressed or snapped into a metal shell (a polyethylene sandwich construction)

For a given size of acetabulum, the maximum diameter of the articulating surfaces is limited by the comparatively thick wall of the cup, which is unavoidable for this type of modular construction. This limitation is important, since the benefits of a large articulation, notably increased stability and range of motion, are widely recognised. In particular, it would be impossible to make a modular cup of this type for a femoral surface replacement, since it would require excessive reaming of either the acetabulum or the femoral head.

A modular construction also brings other disadvantages. Micromotion at the interface between the modular components can lead to wear. Assembly of taper-fit ceramic components requires care, since misalignment can lead to fracture of the ceramic.
Monoblock cups offer simplicity and safety, and are therefore an attractive option for surgeons.

The loosening observed with early monoblock ceramic cups was, in part, attributable to the use of screw-in fixation. However, it has been suggested that an additional reason for loosening is the poor osseointegration potential of the bioinert alumina material. In contrast, metals such as titanium or titanium alloys are widely recognised to be a good choice for bone attachment surfaces of orthopaedic implants.

One means of applying a bone ingrowth surface on to a ceramic cup is by depositing a coating of metal onto the ceramic, for instance, by plasma spraying. This has been described in patent US6319285. However, the plasma sprayed metal coating is not bonded to the ceramic, and the ceramic surface has to be roughened to obtain adequate adhesion of the coating. This will weaken the ceramic.

EP 0202141 discloses a femoral head prosthesis in which a metal support element is permanently secured to a ceramic bearing element.

US 5716414 discloses an acetabulr prosthesis having a ceramic bearing seated within a metal shell.

WO98/42390 discloses a method of forming an oxide coating on an article formed from zirconium, or zirconium alloy.

### STATEMENT OF INVENTION

According to the present invention there is provided a acetabular component of a hip prosthesis comprising a metal support element and a ceramic bearing element, characterised in that the ceramic bearing element is permanently bonded to the metal support element, and in that the surface of the ceramic bearing element that is bonded to the metal support element is smooth.

Preferably, the metal support element and the ceramic bearing element are provided with respective substantially hemispherical bearing surfaces, the metal support element and the ceramic bearing element being permanently bonded over substantially the entire area of the hemispherical bearing surfaces.

In a preferred embodiment the ceramic bearing element comprises a ceramic liner forming the articulating surface, which is fixed to a support element comprising a metal shell which forms or supports the surface apposed to the bone. The metal shell could be fabricated by turning, spinning, casting, forging, or pressing, and bonded to the ceramic cup, preferably by diffusion bonding or by active alloy brazing. This would enable a good bond to be created even on a very smooth ceramic surface, thus maximising the strength of the cup. Other possible methods for bonding the metal shell to the ceramic inlay include gluing, or brazing. The shell could be textured, or coated with a rough or porous coating, to provide a bone ingrowth surface. Alternatively, the shell could be made from an open cell porous metal. In addition, the surface of the shell could be coated with hydroxyapatite or other coating or treatment to promote and enhance bone ingrowth.

The shell could be constructed in a range of sizes to form part of a resurfacing hip replacement.

One of the difficulties with such a construction is the difference in thermal expansion between the metal shell and the ceramic liner. Bonding methods such as diffusion bonding or brazing involve heating the components, and on subsequent cooling to room temperature, the difference in contraction between the two components can lead to high interface stresses and cracking, which can propagate into the ceramic. There are several ways of overcoming this:
1. The metal component can be made from an alloy in which the thermal expansion coefficient is matched to the ceramic. For instance, an alloy of titanium and tantalum and/or niobium could be used with alumina.
2. The metal backing can be formed in a patchwork pattern with gaps to accommodate the differential shrinkage on cooling.
3. The ceramic can be bonded to a shell formed from an open-cell porous metal. The metal-ceramic bond will be in the form of discrete patches. The low modulus of a trabecular structured metal will reduce the stresses due to differential shrinkage.

The preferred method of joining the metal shell to the ceramic component is diffusion bonding. A problem with this technique, particularly for bonding titanium to alumina is the formation of brittle intermetallics and oxides, such as TiAl and TiO₂.

One method for overcoming this is to interpose an intermediate layer between the metal and the ceramic. This interlayer can act as a diffusion barrier to minimise the formation of brittle phases at the interface. In addition, a soft metallic interlayer can reduce thermal stresses of a titanium-ceramic joint by plastic deformation. Interlayers proposed for titanium-porcelain dental implants include silicon nitride, niobium, tantalum, and gold. This interlayer can be applied in the form of a foil, or can be deposited on the metal shell or ceramic inlay prior to assembly and bonding.

Preferably, the ceramic bearing element comprises but is not limited to, alumina, zirconia, zirconia toughened alumina, silicon carbide and/or silicon nitride.

Preferably, the metal support element comprises any biocompatible metal, including pure titanium, titanium alloy (such as titanium-aluminium-vanadium, titanium-niobium, titanium-tantalum), cobalt alloys such as cobalt chromium molybdenum, stainless steel, tantalum and/or zirconium.

For a better understanding of the present invention, and to show more clearly how it may be carried into effect, reference will now be made, by way of example, to the accompanying drawings, in which, Figures 1 to 5 represent embodiments of the invention. Figures 6 and 7 illustrate prostheses that are outside the scope of the invention and are included for information only.

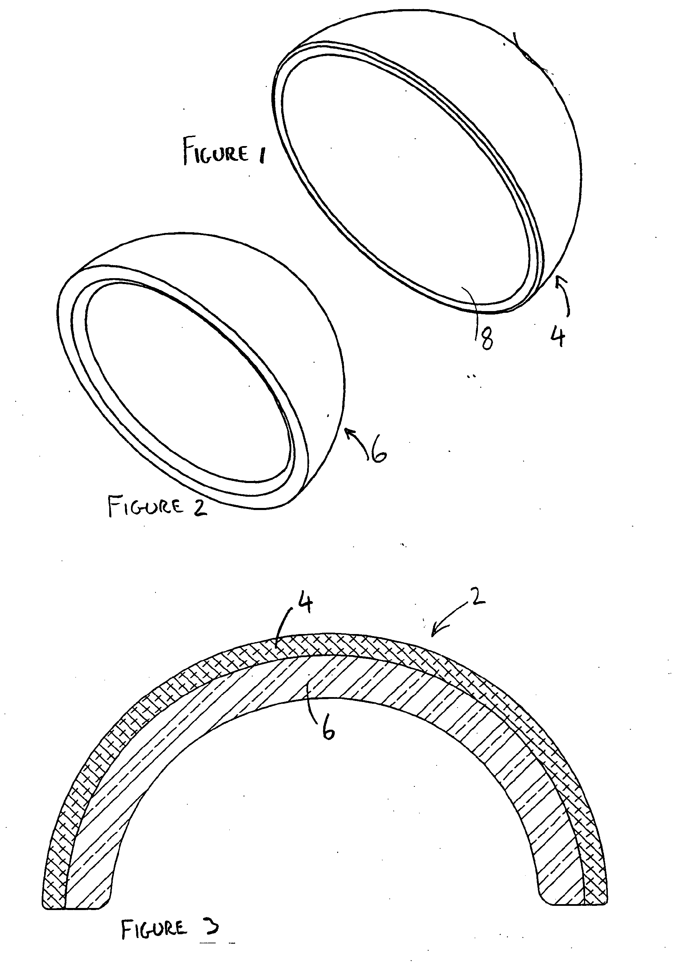

Figure 1 is a perspective view of an acetabular cup;
Figure 2 is a perspective view of a ceramic bearing;
Figure 3 is a cross-section through an acetabular component formed from the acetabular cup and ceramic bearing of Figures 1 and 2.

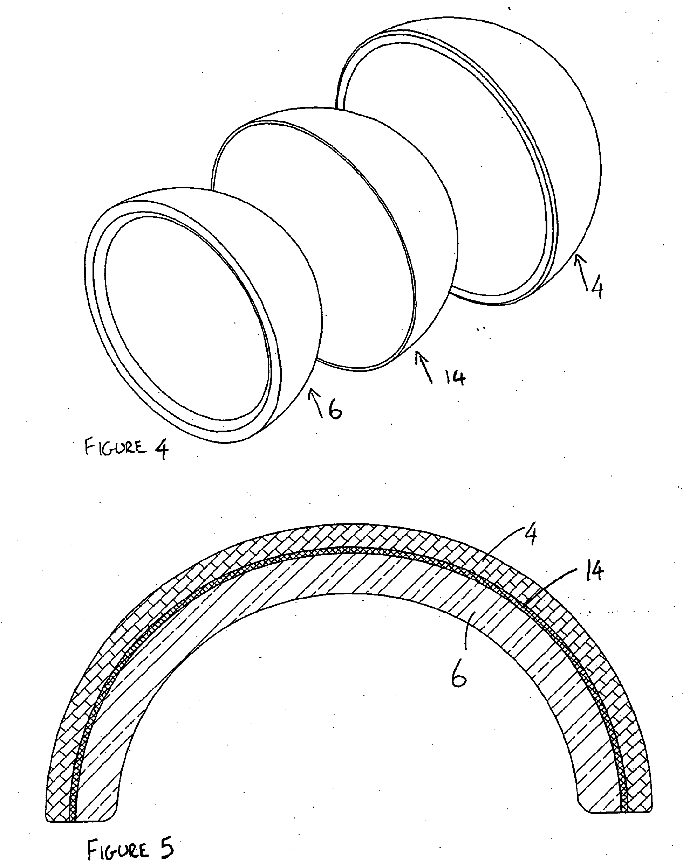

Figure 4 is an exploded view of an acetabular component comprising an acetabular cup, an intermediate element and a ceramic bearing;
Figure 5 is a cross-section through the acetabular component of Figure 4 in an assembled condition;

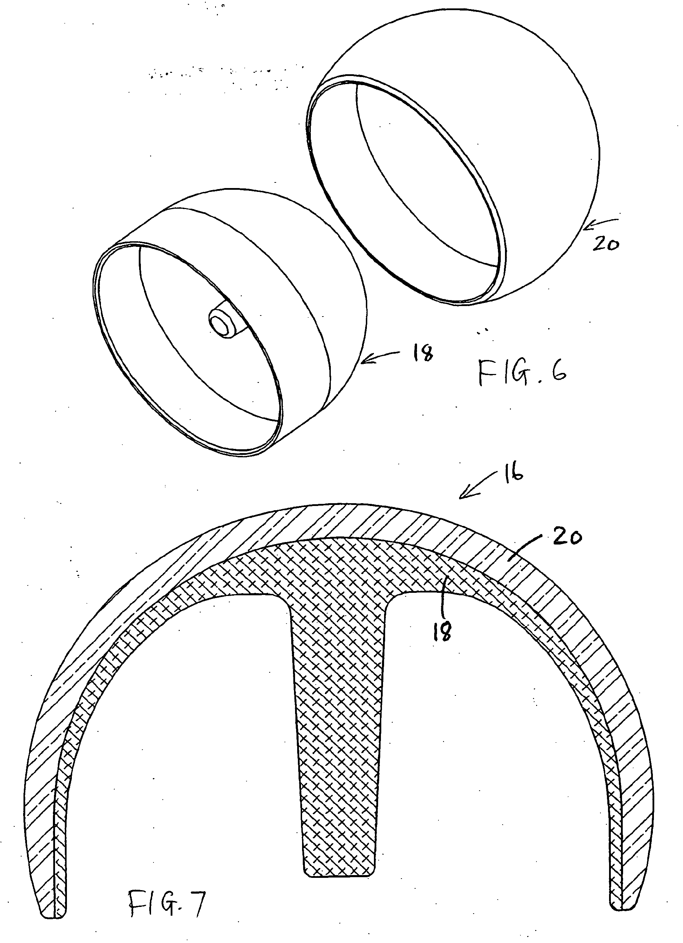

Figure 6 is an exploded view of a femoral head resurfacing prosthesis that is outside the scope of the present invention; and
Figure 7 is a femoral head resurfacing prosthesis in an assembled condition.

Referring to Figures 1 to 3, an acetabular component 2 of a hip prosthesis comprises a support element in the form of a substantially hemispherical metal shell 4 into which is permanently fixed a substantially hemispherical bearing element comprising a ceramic liner 6.

The metal shell 4 has a substantially hemispherical inner surface 8 and a substantially hemispherical outer surface 12, and the ceramic liner 6 has a substantially hemispherical outer surface 10 which conforms in shape very closely to the shape of the inner surface 8 of the shell 4, to facilitate fixing of the liner 6 into the shell 4.

The liner 6 may be fixed to the shell 4 by, for example, diffusion bonding, active alloy brazing, or gluing. In a preferred process, the inner surface 8 and outer surface 10 are treated with flux before being pushed together. Then the entire acetabular component 2 is heated, and brazing material is introduced into the gap between the inner surface 8 and outer surface 10, thereby brazing the liner 6 into the shell 4.

Once assembled, the acetabular component 2 is fitted in a reamed opening (not shown) formed in the acetabulum of a patient. To facilitate the connection between the shell 4 and the acetabulum, the outer surface 12 of the shell 4 could be coated with hydroxyapatite or another coating or treatment which promotes and enhances bone ingrowth. Furthermore, the shell 4 could be made entirely from a porous material and could for example have the form of a rigid mesh or open cell structure which facilitates bone ingrowth.

The inner surface 8 of the shell 4 could be of slightly smaller radius than the outer surface 10 of the liner 6, so that at room (or body) temperature, the liner 6 could not be fitted into the shell 4. The shell 4 is heated, causing it to expand to the point where the liner 6 will fit within the shell 4. The assembly is then allowed to cool, such that the shell 4 grips the liner 6 and provides a permanent connection of the liner 6 within the shell 4. This technique, could be used in association with another fixing technique such as diffusion bonding or brazing. The connection could even be enhanced by use of adhesive, provided that the adhesive could withstand the temperatures required to expand the shell 4. A particular advantage of using a heat shrink technique is that once the acetabular component 2 has cooled, it can be arranged such that the liner 6 is under a compressive load applied by the shell 4. This will greatly enhance the ability of the ceramic liner 6 to withstand cracks and thereby extend its service life, and its ability to withstand impact loading.

Referring to Figures 4 and 5, in an alternative embodiment, an intermediate element in the form of a soft metallic interlayer 14 is interposed between the shell 4 and liner 6. The interlayer 14 is applied in the form of a foil which is disposed over the liner 6, before it is fixed within the shell 4. Alternatively, the interlayer 14 could be deposited on the shell 4 or on the liner 6 prior to assembly and bonding. The interlayer 14 improves adhesion, accommodates discontinuities and manufacturing tolerances in the mating surfaces and/or provides a degree of shock absorption between the shell 4 and liner 6.

Referring to Figures 6 and 7, a femoral head resurfacing prosthesis that is outside the scope of the present invention comprises a support element in the form of a metal cap 18 which is fixed to the end of the resected femur (not shown), and a ceramic bearing element 20 which is fixed over the cap 18 using the techniques described in relation to the embodiment of the invention of Figures 1 to 3. The femoral head prosthesis could also be assembled using an intermediate layer such as the soft metallic interlayer 14 of the embodiment of Figures 4 and 5.

## Claims

1. An acetabular component (2) of a hip prosthesis comprising a metal support element (4) and a ceramic bearing element (6), **characterised in that** the ceramic bearing element (6) is permanently bonded to the metal support element (4), and **in that** the surface (10) of the ceramic bearing element (6) that is bonded to the metal support element (4) is smooth.

2. An acetabular component (2) as claimed in claim 1, in which the support element (4) is bonded to the bearing element (6) by diffusion bonding.

3. An acetabular component (2) as claimed in claim 1, in which the support element (4) is bonded to the bearing element (6) by brazing.

4. An acetabular component (2) as claimed in claim 3, in which the support element (4) is bonded to the bearing element (6) by active alloy brazing.

5. An acetabular component (2) as claimed in claim 1, in which the support element (4) is bonded to the bearing element (6) by adhesive.

6. An acetabular component (2) as claimed in any one of the preceding claims, in which a surface (12) of the support element (4) has a rough and/or porous coating.

7. An acetabular component (2) as claimed in claim 6, in which the support element (4) is coated with hydroxyapatite.

8. An acetabular component (2) as claimed in any one of the preceding claims, in which the support element (4) is made from a porous material.

9. An acetabular component (2) as claimed in any one of the preceding claims, in which an intermediate element (14) is disposed between the support element (4) and the bearing element (6).

10. An acetabular component (2) as claimed in claim 9, in which the intermediate element (14) is a soft metallic interlayer (14).

11. An acetabular component (2) as claimed in claim 9 or 10, in which the intermediate element (14) comprises silicon nitride, niobium, tantalum and/or gold.

12. An acetabular component (2) as claimed in any one of claims 9 to 11, in which the intermediate element (14) 5 comprises a foil.

13. An acetabular component (2) as claimed in any one of claims 9 to 12, in which the intermediate element (14) is deposited onto the support element (4) and/or the bearing element (6) prior to bonding these elements together.

14. An acetabular component (2) as claimed in any one of the preceding claims, in which the bearing element (6) comprises alumina, zirconia, zirconia toughened alumina, silicon carbide and/or silicon nitride.

15. An acetabular component (2) as claimed in any one of the preceding claims, in which the support element (4) comprises titanium, titanium alloy or a cobalt alloy.

16. An acetabular component (2) as claimed in any one of the preceding claims, in which the support element (4) comprises a metal shell (4), and the ceramic bearing element (6) is permanently bonded into the metal shell (4).

17. An acetabular component (2) as claimed in any one of the preceding claims, in which the metal support element (4) and the ceramic bearing element (6) are provided with respective substantially hemispherical bearing surfaces, the metal support element (4) and the ceramic bearing element (6) being permanently bonded over substantially the entire area of the hemispherical bearing surfaces.

## Patentansprüche

1. Pfannenkomponente (2) einer Hüftprothese, umfassend ein Metallstützteil (4) und ein keramisches Lagerteil (6), **dadurch gekennzeichnet, dass** das keramische Lagerteil (6) permanent mit dem Metallstützteil (4) verbunden ist, und dass die Oberfläche (10) des keramischen Lagerteils (6), welches mit dem Metallstützteil (4) verbunden ist, glatt ist.

2. Pfannenkomponente (2) nach Anspruch 1, wobei das Stützelement (4) durch Diffusionsbindung mit dem Lagerteil (6) verbunden ist.

3. Pfannenkomponente (2) nach Anspruch 1, wobei das Stützteil (4) durch Hartlöten mit dem Lagerteil (6) verbunden ist.

4. Pfannenkomponente (2) nach Anspruch 3, wobei das Stützelement (4) durch aktives Legierungshartlöten mit dem Lagerteil (6) verbunden ist.

5. Pfannenkomponente (2) nach Anspruch 1, wobei das Stützteil (4) durch Kleben mit dem Lagerteil (6) verbunden ist.

6. Pfannenkomponente (2) nach irgendeinem der vorhergehenden Ansprüche, wobei eine Oberfläche (12) des Stützelements (4) eine raue und/oder poröse Beschichtung besitzt.

7. Pfannenkomponente (2) nach Anspruch 6, wobei das Stützelement (4) mit Hydroxyapatit beschichtet ist.

8. Pfannenkomponente (2) nach irgendeinem der vorhergehenden Ansprüche, wobei das Stützelement (4) aus einem porösen Material hergestellt ist.

9. Pfannenkomponente (2) nach irgendeinem der vorhergehenden Ansprüche, wobei zwischen dem Stützteil (4) und dem Lagerteil (6) ein Zwischenteil (14) angeordnet ist.

10. Pfannenkomponente (2) nach Anspruch 9, wobei das Zwischenteil (14) eine weiche metallische Zwischenschicht (14) ist.

11. Pfannenkomponente (2) nach Anspruch 9 oder 10, wobei das Zwischenteil (14) Siliziumnitrid, Niob, Tantal und/oder Gold aufweist.

12. Pfannenkomponente (2) nach irgendeinem der Ansprüche 9 bis 11, wobei das Zwischenteil (14) eine Folie umfasst.

13. Pfannenkomponente (2) nach irgendeinem der Ansprüche 9 bis 12, wobei das Zwischenteil (14) auf dem Stützteil (4) und/oder dem Lagerteil (6) vor dem Verbinden dieser Teile miteinander angeordnet wurde.

14. Pfannenkomponente (2) nach irgendeinem der vorhergehenden Ansprüche, wobei das Lagerteil (6) Aluminiumoxid aufweist, Zirkonoxid, Zirkonoxidgehärtetes Aluminiumoxid, Siliziumcarbid und/oder Siliziumnitrid.

15. Pfannenkomponente (2) nach irgendeinem der vorhergehenden Ansprüche, wobei das Stützteil (4) Titan aufweist, Titanlegierung oder eine Kobaltlegierung.

16. Pfannenkomponente (2) nach irgendeinem der vorhergehenden Ansprüche, wobei das Stützteil (4) einen Metallmantel (4) umfasst und das keramische Lagerteil (6) permanent in den Metallmantel (4) eingebaut ist.

17. Pfannenkomponente (2) nach irgendeinem der vorhergehenden Ansprüche, wobei das metallische Stützelement (4) und das keramische Lagerteil (6) mit entsprechenden, im Wesentlichen halbkugelförmigen Lagerflächen versehen sind, und das Metallstützteil (4) und das keramische Lagerteil (6) im Wesentlichen über die gesamte Fläche der halbkugelförmigen Lagerflächen miteinander permanent verbunden sind.

## Revendications

1. Composant acétabulaire (2) d'une prothèse de hanche comprenant un élément métallique de support (4) et un élément céramique (6) de palier, **caractérisé en ce que** l'élément céramique (6) de palier est lié de manière permanente à l'élément métallique de support (4), et **en ce que** la surface (10) de l'élément céramique (6) de palier qui est liée à l'élément métallique de support (4) est lisse.

2. Composant acétabulaire (2) selon la revendication 1, dans lequel l'élément de support (4) est lié à l'élément de palier (6) par liaison par diffusion.

3. Composant acétabulaire (2) selon la revendication 1, dans lequel l'élément de support (4) est lié à l'élément de palier (6) par brasage.

4. Composant acétabulaire (2) selon la revendication 3, dans lequel l'élément de support (4) est lié à l'élément de palier (6) par brasage d'un alliage actif.

5. Composant acétabulaire (2) selon la revendication 1, dans lequel l'élément de support (4) est lié à l'élément de palier (6) par un adhésif.

6. Composant acétabulaire (2) selon l'une quelconque des revendications précédentes, dans lequel une surface (12) de l'élément de support (4) a un revêtement rugueux et/ou poreux.

7. Composant acétabulaire (2) selon la revendication 6, dans lequel l'élément de support (4) est revêtu d'hydroxyapatite.

8. Composant acétabulaire (2) selon l'une quelconque des revendications précédentes, dans lequel l'élément de support (4) est formé d'un matériau poreux.

9. Composant acétabulaire (2) selon l'une quelconque des revendications précédentes, dans lequel un élément intermédiaire (14) est disposé entre l'élément de support (4) et l'élément de palier (6).

10. Composant acétabulaire (2) selon la revendication 9, dans lequel l'élément intermédiaire (14) est une couche intermédiaire métallique tendre (14).

11. Composant acétabulaire (2) selon la revendication 9 ou 10, dans lequel l'élément intermédiaire (14) comprend du nitrure de silicium, du niobium, du tantale et/ou de l'or.

12. Composant acétabulaire (2) selon l'une quelconque des revendications 9 à 11, dans lequel l'élément intermédiaire (14) est une feuille.

13. Composant acétabulaire (2) selon l'une quelconque des revendications 9 à 12, dans lequel l'élément intermédiaire (14) est déposé sur l'élément de support (4) et/ou l'élément de palier (6) avant la liaison de ces éléments l'un à l'autre.

14. Composant acétabulaire (2) selon l'une quelconque des revendications précédentes, dans lequel l'élément de palier (6) comprend de l'alumine, de la zircone, de l'alumine renforcée par de la zircone, du carbure de silicium et/ou du nitrure de silicium.

15. Composant acétabulaire (2) selon l'une quelconque des revendications précédentes, dans lequel l'élément de support (4) contient du titane, un alliage de titane ou un alliage de cobalt.

16. Composant acétabulaire (2) selon l'une quelconque des revendications précédentes, dans lequel l'élément de support (4) comporte une coquille métallique (4), et l'élément céramique de palier (6) est lié de façon permanente dans la coquille métallique (4).

17. Composant acétabulaire (2) selon l'une quelconque des revendications précédentes, dans lequel l'élément métallique de support (4) et l'élément céramique de palier (6) sont munis de surfaces pratiquement hémisphériques respectives de palier, l'élément métallique de support (4) et l'élément céramique de palier (6) étant liés de façon permanente sur pratiquement toute l'étendue des surfaces hémisphériques de palier.
